# EUROPEAN PATENT APPLICATION

(11) **EP 2 283 811 A1**
(43) Date of publication of application: **16.02.2011**
(21) Application number: 10176222.7
(22) Date of filing: 19.10.2005
(51) Int. Cl.: A61K 9/00, A61K 9/20, A61K 31/445, C07D 211/32

(54) **Solid pharmaceutical composition comprising donepezil hydrochloride**

(30) Priority: 19.10.2004 DE 102004051055
(62) Divisional of application: 08020395.3
(71) Applicant: Krka Tovarna Zdravil, D.D., Novo Mesto, 8501 Novo Mesto (SI)
(72) Inventor: Vrbinc, Miha, 8000, Novo mesto (SI); Kotar-Jordan, Berta, 8311, Kostanjevica na Krki (SI); Zupet, Rok, 1211, Ljubljana (SI); Smrkolj, Matej, 1420, Trbovlje (SI)
(74) Representative: UEXKÜLL & STOLBERG

(57) **Abstract**

The invention relates to a solid pharmaceutical composition comprising donepezil hydrochloride hydrate and a process for its preparation. In particular it relates to a composition and a process wherein the donepezil hydrochloride retains its polymorphic form and is therefore highly stable against conversion into other polymorphic forms.

## Description

The invention relates to a solid pharmaceutical composition comprising donepezil hydrochloride and a process for its preparation. In particular it relates to a composition and a process wherein the donepezil hydrochloride retains its polymorphic form and is therefore highly stable against conversion of polymorphic forms.

Donepezil hydrochloride, polymorphic forms thereof and pharmaceutical compositions comprising donepezil hydrochloride are known.

WO 97/46527 describes a method for the preparation of the polymorphic forms I to V and of the amorphous form of do-nepezil hydrochloride. The polymorphs are characterised by characteristic peaks in the powder X-ray diffraction pattern and wave numbers (cm⁻¹) of infrared absorption spectra in potassium bromide. Different methods for producing the form I of donepezil hydrochloride are described. However, no specific solid pharmaceutical compositions are disclosed, let alone data on the stability of donepezil hydrochloride, either in the amorphous or in the different crystal forms, when incorporated in such a composition.

EP-A-1 086 706 discloses compositions of donepezil which have been stabilised against the effect of light and heat by the addition of an organic acid. It is also shown by examples that the use of organic acids results, after storing the composition at elevated temperatures, in the production of less impurities in comparison to the use of hydrochloric acid.

EP-A-1 378 238 describes pharmaceutical compositions which comprise donepezil hydrochloride in amorphous form. It is further discussed in this document that it is not an easy task to reproducibly prepare compositions including the desired polymorphic form of donepezil hydrochloride since it is showing polymorphism and since similar procedures may nevertheless lead to different crystalline forms.

Thus, the prior art does not deal with the problem of avoiding a conversion of the polymorphic form of donepezil hydrochloride when processing it to the desired solid composition. Further, the prior art also does not address the problem of stabilising the polymorphic form of donepezil hydrochloride during the shelf-life of a corresponding solid composition.

These problems are surprisingly solved by the present invention.

The solid pharmaceutical composition according to the invention comprises donepezil hydrochloride and excipients, and has a water content of 3 to 10 %, preferably 4 to 7 % and more preferably 5 to 6 % by weight as determined by Karl Fischer (test performed according to Ph. Eur. 2.5.12, e.g. on a Karl Fischer titrator Metrohm 7012 KF Titrino). The donepezil hydrochloride is used in form of a hydrate, preferably in the form of the monohydrate.

It has unexpectedly been found out that it is critical to control the water content of the composition to lie in the above range in order to avoid the undesired conversion of the specific polymorphic form of donepezil hydrochloride in the composition to other hydrated or anhydrous polymorphic forms. In particular the conversion of hydrated forms into anhydrous forms is a problem with conventional compositions.

The donepezil hydrochloride is present in the compositions of the invention in crystalline form. More preferably the donepezil hydrochloride is present in one of various polymorphic forms, in particular as polymorph I or IV. These polymorphic forms of donepezil hydrochloride as well as the preparation thereof are disclosed in WO 97/46527 the contents of which is incorporated herein by reference. The compositions of the present invention thus preferably contain polymorph I and/or polymorph IV of donepezil hydrochloride in the form of a hydrate.

A composition is particularly preferred wherein the donepezil hydrochloride is donepezil hydrochloride of polymorphic form I and in particular the monohydrate of polymorphic form I. Such a composition has been found to be very stable against undesired changes to other polymorphic form(s) of the active ingredient.

In a further preferred embodiment the composition according to the invention is such that the average particle size of the donepezil hydrochloride is 5 to 300 µm, preferably 10 to 150 µm. The average particle size is determined by laser method on 10 Malvern Mastersizer.

Donepezil hydrochloride hydrate of form I or form IV is preferably prepared by suspending donepezil hydrochloride in a solvent. Alternatively donepezil hydrochloride can be prepared from donepezil base and hydrochloric acid. Preferably a mixture of methanol and water is used as the solvent. Optionally other alcohols, such as ethanol or isopropanol, or mixtures of alcohols with water can be used. The formation of donepezil hydrochloride hydrate form I or form IV depends upon the water content in the solvent mixture. The suspension is heated until the donepezil hydrochloride has completely dissolved in the solvent. Optionally this solution can be filtered trough 1 µm filtration cartridge. The solution of donepezil hydrochloride is then cooled to approximately 40°C. Donepezil hydrochloride hydrate of form I or form IV is precipitated from this solution by the addition of isopropyl ether, isopropyl acetate, ethyl acetate, butyl acetate, isobutyl methyl ketone, tert.-butyl methyl ether or heptane.

It is further preferred that the particle sizes of the other excipients used in the pharmaceutical compositions of the present invention are within the range of D90<500 µm, preferably D90<350 µm, in order to ensure homogeneity of the compression mixture and homogeneous granulation and compression. D90 means that at least 90 % by volume or weight of the particles have a particle size below the specified value.

The excipients present in the composition according to the invention can be diluents such as microcrystalline cellulose, powdered cellulose, lactose (anhydrous or preferably monohydrate), compressible sugar, fructose, dextrates, other sugars such as mannitol, sorbitol, lactitol, sacharose or a mixture thereof, siliconised microcrystalline cellulose, calcium hydrogen phosphate, calcium carbonate, calcium lactate or mixtures of diluents. Preferably, the excipients include at least one diluent, selected from microcrystalline cellulose and lactose monohydrate.

The composition according to the invention can also comprise binders, such as polyvinyl pyrrolidone, microcrystalline cellulose, hydroxyethyl cellulose, hydroxypropyl cellulose, low-substituted hydroxypropyl cellulose, hydroxypropylmethyl cellulose or other cellulose ethers, starch, pregelatinised starch, or polymethacrylate or mixtures of binders. It is preferred that the excipients include at least one binder selected from hydroxypropyl cellulose, starch, in particular corn starch, pregelatinised starch and hydroxypropylmethyl cellulose. Low-substituted hydroxypropyl cellulose is hydroxypropyl cellulose comprising from 5 to 16 % by weight of hydroxypropoxy groups. Suitable low-substituted hydroxypropyl celluloses are commercially available from Shin-Etsu Chemical Co., Ltd. under the trade names LH-11, LH-20, LH-21, LH-22, LH-30, LH-31 and LH-32.

Further, disintegrants can also be present, such as starch, e.g. pregelatinised starch, corn starch or others, sodium starch glycolate, crospovidone, microcrystalline cellulose, carboxymethylcellulose sodium, polacrilin potassium, low-substituted hydroxypropyl cellulose or mixtures thereof. If used as a disintegrant, microcrystalline cellulose is preferably used in an amount of 5 to 15 % by weight. It is preferred that the excipients include at least one disintegrant selected form starch, crospovidone and low-substituted hydroxypropyl cellulose.

The disintegrants can be added to the other excipients according to the process used in the state of the art, either in the process of granulating and/or in the preparation of the compression mixture.

Further, lubricants can also be present as excipients, such as stearic acid, magnesium stearate, calcium stearate, sodium laurylsulphate, hydrogenated vegetable oil, hydrogenated castor oil, sodium stearyl fumarate, talc, or macrogols or mixtures thereof. It is preferred that the excipients include at least one lubricant selected from hydrogenated castor oil, talc and magnesium stearate.

A composition is particularly preferred which comprises:
(A) 1 to 50 %, preferably 1 to 40 %, more preferably 1 to 30 % by weight of donepezil hydrochloride,
(B) 1 to 90 %, preferably 20-85% by weight of diluent,
(C) 1 to 90 %, preferably 10-40% by weight of binder,
(D) 1 to 40 %, preferably 10-40% by weight of disintegrant, and optionally
(E) 0.1 to 10%, preferably 0.1-5% by weight of lubricant.

Even more preferred are compositions comprising the above defined preferred excipients. If not indicated otherwise all percentages given herein are by weight based on the total weight of the composition.

Investigations have also shown that the achieving of a stable composition in terms of avoiding conversion of the polymorphic form of donepezil hydrochloride used is surprisingly possible by a rather small difference of the water contents of the donepezil hydrochloride and the excipients. Thus the water content of the active ingredient and the various excipients used in the compositions of the present invention is adjusted in such a way that a migration of water from the excipients to the donepezil hydrochloride or vice versa is prevented.

In a preferred embodiment the invention relates to a solid pharmaceutical composition comprising:
(a) donepezil hydrochloride, and
(b) excipents, which are present in the composition in the amount of more than 11 % (e.g. more than 11 to 99 %), preferably more than 15% (e.g. more than 15 to 99 %), more preferably more than 20 % (e.g. more than 20 to 99 %) based on the total composition weight, and
(c) excipients, which are present in the composition in the amount of less than 11 % (e.g. 0.1 to less than 11 %), preferably less than 15 % (e.g. 0.1 to less than 15 %), more preferably less than 20% (e.g. 0.1 to less than 20 %) based on the total composition weight,
wherein the water content of excipients (b), in % by weight, minus the water content of active ingredient (a), in % by weight, is less than 4.0% (by weight), preferably less than 3.0% by weight, most preferably less than 2.0 % by weight, determined by Karl Fisher (test performed according to Ph. Eur. 2.5.12, e.g. on a Karl Fischer titrator Metrohm 7012 KF Titrino). Excipient (c) may be absent even though it is preferred that excipient (c) is present.

Preferred as excipients (b) are lactose monohydate, microcrystalline cellulose, powdered cellulose, dextrates (hydrated), lactitol (hydrated), siliconised microcrystalline cellulose, sacharose, calcium hydrogen phosphate, calcium carbonate, calcium lactate, or mixtures thereof.

Preferred as excipients (c) are polyvinyl pyrrolidone, carboxymethylcellulose sodium, polacriclin potassium, starch, sodium starch glycolate, hydroxyethyl cellulose, hydroxypropyl cellulose, hydroxypropylmethyl cellulose or other cellulose ethers, polymethacrylate, crospovidone, stearic acid, magnesium stearate, calcium stearate, sodium laurylsulphate, hydrogenated vegetable oil, hydrogenated castor oil, sodium stearyl fumarate, talc, macrogols, or mixtures thereof.

The donepezil hydrochloride (a) is preferably a donepezil hydrochloride hydrate, more preferably a hydrate of polymorphic form IV and in particular of form I.

It is assumed that the small difference in terms of water content of the ingredients of this composition effectively prevents a migration of water from the donepezil hydrochloride to the excipients and vice versa, thereby stabilising donepezil hydrochloride in the form originally used, i.e. preventing a conversion e.g. of donepezil hydrochloride polymorphic form I into another polymorphic form.

The composition according to the invention is preferably in form of a coated or uncoated tablet, e.g. fast disintegrating tablet or orally disintegrating tablet, a capsule or in the form of pellets. The composition can also take the form of a powder mixture, of a granulate or of mini tablets filled in capsules. An immediate release composition is preferable.

The composition according to the invention can be prepared by a process which comprises mixing and processing donepezil hydrochloride and excipients to the desired composition.

As the composition is characterised by a specific water content as given above, its components, in particular their amount and their water content, and the way of processing them to the composition are selected such that the desired water content is achieved in the composition. This can in particular be effected by wetting a part of or all excipients with water, which can be achieved in a separate step or as part of the production process, for example in the course of a granulation step with water or aqueous granulation liquids.

Due to the problem that in particular donepezil hydrochloride in monohydrate form, e.g. donepezil hydrochloride of form I, can transform into an anhydrous form, the choice of the process and of the excipients is critical to achieve the desired water content of the composition or the desired small difference as to water content of donepezil hydrochloride and excipients which result in a highly stable final product.

A wetting of excipients can be performed in conventional granulation equipment by spraying of purified water into the excipients by conventional techniques. Wetting can also be effected by direct addition of purified water onto a mixture of excipients during a mixing operation in a proper mixing device, e.g. high-shear mixer.

The mixing of excipients or of excipients with donepezil hydrochloride may be effected in conventional devices used for mixing of powders, e.g. motionless (passive) mixers, fluidized bed, diffusion, biconic diffusion, biconic, turbula, cubic, planetary, Y-, V-shaped or high-shear mixers.

In case the composition is defined by the difference as to the water contents of donepezil hydrochloride and excipients, then their respective water content is to be adjusted accordingly in the process for its preparation.

The process for preparing the composition according to the invention can be carried out as a granulation process or a direct compression process.

In a preferred embodiment, the granulation process comprises
(i) granulating a mixture of excipients using water as granulation liquid to give a granulate,
(ii) adding donepezil hydrochloride and excipients to the granulate to give a compression mixture,
(iii) compressing the compression mixture to the desired form, and
(iv) optionally applying a coating.

Thus, in this embodiment, a granulate is prepared in step (i) which does not include the active ingredient donepezil hydrochloride.

In another preferred embodiment, the granulation process comprises
(i') granulating a mixture of donepezil hydrochloride and excipients using water as granulation liquid to give a granulate,
(ii') adding excipients to the granulate to give a compression mixture,
(iii') compressing the compression mixture to the desired form, and
(iv') optionally applying a coating.

Thus, in this embodiment a granulate is prepared in step (i') which includes active ingredient.

The excipients used in steps (ii) and (ii'), respectively, can be the same or different excipients as used in steps (i) and (i'), respectively.

It has been found preferable that the temperature of the granulate in a granulation process does not exceed 50°C during the granulating step. It is assumed that this is useful to prevent undesired changes of the polymorph used to other forms as may occur when using high temperatures, in particular to remove granulation liquid after a granulating process.

In particular the temperature used when drying the wet granulate should be low, e.g. the temperature of the inlet air in a fluid bed dryer should be around 70°C or lower, to ensure that the temperature of the granulate does not exceed 50°C. Use of high temperatures accelerates transformation of a hydrated form to other hydrated or anhydrous forms.

Further, it has also been found beneficial to adjust the water content of the granulate to 0.5 to 2.5 %, preferably 1.0 to 2.0 % by weight (determined as loss on drying, at 85°C, 20 minutes, e.g. with a Mettler Toledo HR73 halogen moisture analyser).

It is also preferred that the water content of the compression mixture is 1.0 to 6.0 %, preferably 1.5 to 5.0 % by weight (determined as loss on drying, at 85°C, 20 minutes, e.g. with a Mettler Toledo HR73 halogen moisture analyser).

For drying the granulation conventional drying devices such as a fluid-bed dryer or drying chambers can be used.

Further, a preferred embodiment of a direct compression process comprises
(i") mixing donepezil hydrochloride and excipients to give a compression mixture,
(ii") compressing the obtained compression mixture to the desired form, and
(iii") optionally applying a coating.

Also in the direct compression process, it is preferred that the water content of the compression mixture is 1.0 to 6.0 %, preferably 1.5 to 5.0 % by weight, (determined as loss on drying, at 85°C, 20 minutes, e.g. with a Mettler Toledo HR73 halogen moisture analyser).

In the above processes according to the invention the compression, in particular to tablets, can be effected using rotary press machines from different manufacturers.

Optionally, the tablets can be coated with conventional materials used for film coating, i.e. as described in Pharmaceutical Coating Technology, 1995, edited by Graham Cole. The film coating formulations preferably contain the following components: polymer(s), plasticizer(s), colourant(s)/opacifier(s), vehicle(s). In the film coating suspension minor quantities of flavours, surfactants and waxes can be used. The majority of the polymers used in film coating are preferably either cellulose derivatives, such as cellulose ethers, or acrylic polymers and copolymers. High molecular weight polyethylene glycols, polyvinyl pyrrolidone, polyvinyl alcohol and waxy materials can also be used.

Typical cellulose ethers are hydroxyethylcellulose, hydroxypropylcellulose, hydroxypropylmethylcellulose, methyl-cellulose. Suitable acrylic polymers include synthetic polymers with diverse functionalities. They may be further modified to enhance swelling and permeability by the incorporation of materials such as water soluble cellulose ethers and starches in order to ensure complete disintegration/dissolution of the film.

Suitable plasticizers for use in the coating materials can be categorized into three groups: polyols (glycerol, propylene glycol, macrogols), organic esters (phthalate esters, dibutyl sebacetate, citrate esters, triacetin), oils/glycerides (castor oil, acetylated monoglycerides, fractionated coconut oil).

Suitable colorants/opacifiers can be classified into several groups: organic dyes and lacquers thereof, inorganic colours, natural colours.

Combination of different materials from each group can be combined in defined ratio. Film coating suspensions can used as ready-to-make preparations that are available on the market.

Film coating dispersion can be prepared by using different solvents (water, alcohols, ketones, esters, chlorinated hydrocarbons, preferably water).

A composition of coating suspension (calculated on dry material) is particularly preferred which comprises:
(A') 1-99% by weight of polymer, preferably 1-95% of polymer,
(B') 1-50% by weight of plasticizer, preferably 1-40% of plasticizer,
(C') 0.1-20% of colorant/opacifier, preferably 0.1-10% of colorant/opacifier.

Conventional equipment can be used for applying a coating, such as a Wurster coating system or conventional coating pans.

It has been found to be beneficial that, the water content of film coated tablets is 3 to 10 % by weight, preferably 4 to 7 % by weight and more preferably 5 to 6 % by weight (determined by the Karl-Fischer method, test performed according to Ph. Eur. 2.5.12, e.g. on a titrator Metrohm 7012 KF Titrino).

It was surprisingly found out that the processes according to the invention effect virtually no undesired conversion of the donepezil hydrochloride used as starting material to other forms. This was in particular shown when using donepezil hydrochloride hydrate, which did not undergo appreciable conversion into other hydrated forms or into other anhydrous forms.

Further, also the final composition showed a surprisingly high stability against undesired conversions of the donepezil hydrochloride, in particular of donepezil hydrochloride hydrate, upon storage.

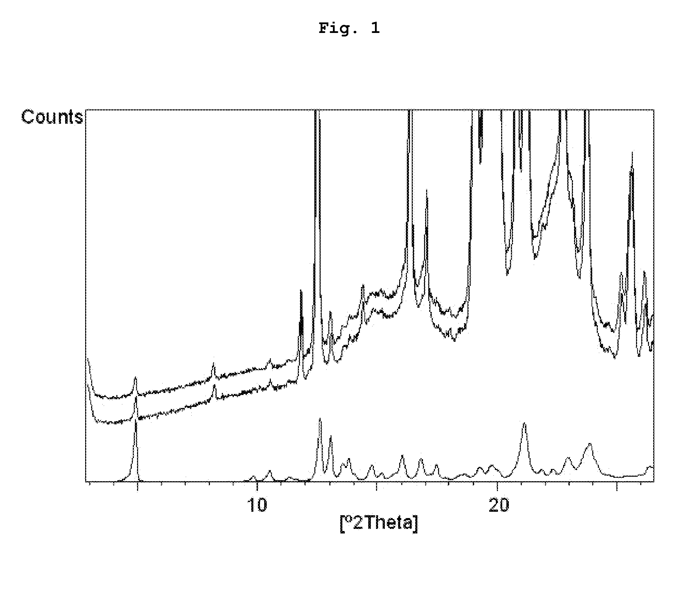

Although accelerated conditions (50°C) were used for stability testing and tablets were not packaged in contact packing material, e.g. blisters, donepezil hydrochloride hydrate remained unchanged. The stability of composition according to the invention was proven by characteristic peaks in the powder X-ray diffraction pattern. The result of this test is shown in Figure 1. Figure 1 shows an X-ray diffraction pattern of tablets comprising donepezil hydrochloride hydrate (upper curve) as well as of pure donepezil hydrochloride hydrate (lower curve). The absence of other diffraction peaks in the tablets pattern indicates the absence of other forms of donepezil hydrochloride.

In the following, further embodiments of the invention are described by numbered paragraphs.
1. Solid pharmaceutical composition comprising donepezil hydrochloride in the form of a hydrate and excipients, and having a water content of 3 to 10 % by weight (determined by Karl Fischer).
2. Composition according to paragraph 1 having a water content of 4 to 7 % by weight (determined by Karl Fischer).
3. Composition according to paragraph 1 or 2, wherein the donepezil hydrochloride is donepezil hydrochloride monohydrate.
4. Composition according to any one of paragraphs 1 to 3, wherein the donepezil hydrochloride is of crystalline form.
5. Composition according to paragraph 4 wherein the donepezil hydrochloride is donepezil hydrochloride of polymorphic form I or IV.
6. Composition according to any one of paragraphs 1 to 5, wherein the excipients include at least one diluent selected from microcrystalline cellulose and lactose monohydrate.
7. Composition according to any one of paragraphs 1 to 6 wherein the excipients include at least one binder selected from hydroxypropyl cellulose, low-substituted hydroxypropyl cellulose, hydroxypropylmethyl cellulose, starch, and pregelatinised starch.
8. Composition according to any one of paragraphs 1 to 7, wherein the excipients include at least one disintegrant selected from starch, crospovidone, and low-substituted hydroxypropyl cellulose.
9. Composition according to any one of paragraphs 1 to 8 comprising as excipient:
   (A) 1 to 50 % by weight of donepezil hydrochloride,
   (B) 1 to 90 % by weight of diluent,
   (C) 1 to 90 % by weight of binder,
   (D) 1 to 40 % by weight of disintegrant, and optionally
   (E) 0.1 to 10 % by weight of lubricant.
10. Composition according to any one of paragraphs 1 to 9 wherein the water content of the donepezil hydrochloride hydrate and the various excipients used in the composition is adjusted in such a way that a migration of water from the excipients to the donepezil hydrochloride or vice versa is prevented.
11. Composition according to paragraph 10 comprising:
   (a) donepezil hydrochloride, and
   (b) excipents, which are present in the composition in the amount of more than 11%, preferably more than 15 %, more preferably more than 20 % based on the total composition weight, and
   (c) excipients, which are present in the composition in the amount of less than 11 %, preferably less than 15%, more preferably less than 20% based on the total composition weight,
   wherein the water content of excipients (b), in % by weight, minus the water content of active ingredient (a), in % by weight, is less than 4.0 % (by weight), preferably less than 3.0 % (by weight), most preferably less than 2.0 % (by weight) (determined by Karl Fischer).
12. Composition according to any one of paragraphs 1 to 11, which is in the form of a tablet.
13. Process for the preparation of the composition according to any one of paragraphs 1 to 12 comprising mixing and processing donepezil hydrochloride and excipients to the desired composition.
14. Process according to paragraph 13 comprising:
   (i) granulating a mixture of excipients using water as granulation liquid to give a granulate,
   (ii) adding donepezil hydrochloride and excipients to the granulate to give a compression mixture,
   (iii) compressing the compression mixture to the desired form, and
   (iv) optionally applying a coating.
15. Process according to paragraph 13 comprising:
   (i') granulating a mixture of donepezil hydrochloride and excipients using water as granulation liquid to give a granulate,
   (ii') adding excipients to the granulate to give a compression mixture,
   (iii') compressing the compression mixture to the desired form, and
   (iv') optionally applying a coating.
16. Process according to paragraph 14 or 15, wherein the temperature of the mixture and of the granulate does not exceed 50°C during the granulating step.
17. Process according to any one of paragraphs 14 to 16, wherein the water content of the granulate is 0.5 to 2.5 %, preferably 1.0 to 2.0 % determined as loss on drying at 85°C, 20 minutes.
18. Process according to any one of paragraphs 14 to 17, wherein the water content of the compression mixture is 1.0 to 6.0 %, preferably 1.5 to 5.0 % determined as loss on drying at 85°C, 20 minutes.
19. Process according to paragraph 13 comprising
   (i') mixing donepezil hydrochloride and excipients to give a compression mixture,
   (ii') compressing the obtained compression mixture to the desired form, and
   (iii') optionally applying a coating.

The present invention will now be further illustrated with reference to Examples and a Figure.

Figure 1 shows an X-ray diffraction pattern of the tablets comprising donepezil hydrochloride hydrate described in Example 4.

### Example 1: Film coated tablets by direct compression process

111.8 g of donepezil hydrochloride hydrate, 1587 g of lactose monohydrate, 857 g of pregelatinised starch and 429 g of corn starch were homogenised in a biconic mixer for 10 minutes at 26 rpm. Finally, 16 g of magnesium stearate were added and the mixing was continued for 3 minutes. The obtained compression mixture was compressed on an automatic rotary press machine, fitted with round punches to give tablet cores, the weight of 10 cores was 300 mg. Subsequently, the cores were coated with the coating suspension, which contains hydroxypropylmethyl cellulose (70 % by weight), polyethylene glycol (5 % by weight), titanium dioxide (20 % by weight), talc (4 % by weight) and iron oxide (1 % by weight) until the average weight of 10 film coated tablets was 308 mg.

The water content of donepezil hydrochloride hydrate was 5.0 % determined by Karl Fischer method, test performed according to Ph. Eur. 2.5.12, on a titrator Metrohm 7012 KF Titrino.

The amount of water in the obtained tablets was 6.0 % by weight, determined by the Karl-Fischer method which was performed according to Ph. Eur. 2.5.12, on a titrator Metrohm 7012 KF Titrino.

### Example 2: Tablets by granulation process

5030 g of microcrystalline cellulose, 1920 g of lactose monohydrate, 840 g of corn starch and 250 g of hydroxypropylcellulose were homogenised in a high-shear mixer. The homogenised mixture was sprayed with purified water and granulated in a high-shear mixer/granulator. The wetted mixture was dried in a fluid-bed dryer using an inlet air temperature of 70°C. The obtained dry granulate was sieved using a sieving machine. Subsequently, 313 g of donepezil hydrochloride hydrate were mixed with the granulate in a biconic mixer. Finally, 45 g of magnesium stearate were admixed to obtain a compression mixture.

The compression mixture was compressed to obtain tablets. Some of these tablets were provided with a film coating as described in Example 1.

The amount of water in the obtained tablets was 5.4 % by weight, determined by the Karl-Fischer method which was performed according to Ph. Eur. 2.5.12, on a titrator Metrohm 7012 KF Titrino.

### Example 3: Tablets by granulation process

5030 g of lactose monohydrate, 1920 g of microcrystalline cellulose and 250 g of hydroxypropylcellulose were homogenised in a high-shear mixer. The homogenised mixture was sprayed with purified water and granulated in a high-shear mixer/granulator. The wetted mixture was dried in a fluid-bed dryer using an inlet air temperature of 70°C. The obtained dry granulate was sieved using a sieving machine. Subsequently, 313 g of donepezil hydrochloride hydrate and 840 g of low-substituted hydroxypropylcellulose were mixed with the granulate in a biconic mixer. Finally, 45 g of magnesium stearate were admixed to obtain a compression mixture.

The compression mixture was compressed to obtain tablets. Some of these tablets were provided with a film coating as described in Example 1.

The amount of water in the obtained tablets was 5.1 % by weight, determined by the Karl-Fischer method which was performed according to Ph. Eur. 2.5.12, on a titrator Metrohm 7012 KF Titrino.

### Example 4: Tablets by granulation process

### (a) Preparation process

3540 g of lactose monohydrate, 1430 g of microcrystalline cellulose, 600 g of corn starch and 180 g of hydroxypropyl cellulose were homogenised in a high-shear mixer. The homogenised mixture was sprayed with purified water and granulated in a high-shear mixer/granulator. The wetted mixture was dried in a fluid-bed dryer using an inlet air temperature of 70 °C. The obtained dry granulate was sieved using a sieving machine. The granulate showed the following particle size distribution:

| **Sieve (µm)** | **Proportion of particles passing through the sieve (weight %)** |
|---|---|
| 71 | 10.2 |
| 125 | 60.9 |
| 250 | 75.5 |
| 500 | 91.8 |
| 710 | 98.4 |
| 1250 | 100.0 |

The water content for dry granulate determined by Karl-Fischer method was 4.8 %. The water determination was performed according to Ph. Eur. 2.5.12, on a titrator Metrohm 7012 KF Titrino.

Subsequently, 223 g of donepezil hydrochloride hydrate were mixed with the granulate in a biconic mixer. Finally, 32 g of magnesium stearate were admixed to obtain a compression mixture.

The compression mixture was compressed into tablets. The average weight of 10 tablets was 200 mg. A portion of the tablets was provided with a coating as described in Example 1.

The amount of water in the obtained tablets was determined to be 5.5 % by the Karl Fischer method which was performed according to Ph. Eur. 2.5.12, on a titrator Metrohm 7012 KF Titrino.

### (b) Stability test of tablets

Accelerated conditions were used for stability testing. Tablets containing donepezil hydrochloride hydrate were stored for 30 days at a temperature of 50°C. Tablets were not packaged in a contact packing material such as blisters. The stability of the composition according to the invention was proven by characteristic peaks in the powder X-ray diffraction pattern. The result of this test is shown in Fig. 1.

Fig. 1 shows an X-ray diffraction pattern of tablets comprising donepezil hydrochloride hydrate. Tablets were prepared by water granulation (upper curve) and stored 30 days at 50°C/dry (middle curve). Donepezil hydrochloride hydrate is shown in the bottom curve. The absence of other diffraction peaks in the tablets pattern stored 30 days at 50°C/dry indicate that there are no other (anhydro and hydro) forms of donepezil hydrochloride present. Donepezil hydrochloride hydrate in the composition according to the invention remains unchanged.

### Example 5: Tablets by granulation process

Example 4 was repeated with the exception that the 180 g of hydroxypropyl cellulose were replaced by 180 g of hydroxypropylmethyl cellulose.

The amount of water in the obtained tablets was 5.3 % by weight, determined by the Karl-Fischer method which was performed according to Ph. Eur. 2.5.12, on a titrator Metrohm 7012 KF Titrino.

### Example 6: Tablets by granulation process

279 g of donepezil hydrochloride hydrate, 4491 g of macrocrystalline cellulose, 1714 g of lactose monohydrate, 223 g of hydroxypropylcellulose and 750 g of low-substituted hydroxypropylcellulose were homogenised in a high shear mixer. The homogenised mixture was sprayed with purified water and granulated in a high-shear mixer/granulator. The wetted mixture was dried in a fluid-bed dryer using an inlet air temperature of 70°C. The obtained dry granulate was sieved using a sieving machine. Finally, 40 g of magnesium stearate were admixed to obtain a compression mixture.

The compression mixture was compressed into tablets. The average weight of the tablets was 250 mg/tablet. Some of these tablets were provided with a coating as described in Example 1.

The amount of water in the obtained tablets was 5.8 % by weight, determined by the Karl-Fischer method which was performed according to Ph. Eur. 2.5.12, on a titrator Metrohm 7012 KF Titrino.

### Example 7: Tablets by granulation process

Example 6 was repeated with the exception that the 4491 g of microcrystalline cellulose and 1714 g of lactose monohydrate were replaced by 4491 g of lactose monohydrate and 1714 g of microcrystalline cellulose. The drying time in the fluid-bed dryer was 19 minutes. The result of particle size distribution for tabletting mixture was as follows:

| **Sieve (pm)** | **Proportion of particles passing through the sieve (weight %)** |
|---|---|
| 71 | 12.2 |
| 125 | 64.5 |
| 250 | 74.9 |
| 500 | 87.8 |
| 710 | 97.9 |
| 1250 | 100.0 |

The amount of water in the tablets was 5.7 % (determined by the Karl-Fischer method performed according to Ph. Eur. 2.5.12, on a titrator Metrohm 7012 KF Titrino).

### Example 8: Preparation of donepezil hydrochloride form I

50.6 g donepezil base were suspended in methanol (300 ml) at room temperature and heated to 60°C to get a clear solution. 11 ml conc. hydrochloric acid were added at 25 to 30°C with measuring pH (3.2 ± 0.2). The solution of donepezil hydrochloride warmed to 40-45°C; it was slowly added to cooled (0-5°C) diisopropyl ether (600 ml) maintaining the temperature at 5-10 °C. The suspension was stirred for another half an hour. The product was filtered and dried in vacuum (30-50 mbar) at a temperature of 30-35°C. The drying was controlled by measuring the water content (Karl Fischer not more than 4.5 %). The yield was 52.66 g (91 %) .

### Example 9: Crystallisation of donepezil hydrochloride form I

50 g donepezil hydrochloride were suspended in methanol (300 ml) and 8 ml of water, heated to 60-65°C to get a clear solution. Then this hot solution was slowly added to cooled (0-5°C) diisopropyl ether (600 ml) maintaining the temperature at 5-10°C. The suspension was stirred for another half an hour. The product was filtered and dried in vacuum (30-50 mbar) at a temperature of 30-35°C. The drying was controlled by measuring the water content as described in Example 8. The yield was 47.39 g (91 %).

### Example 10: Preparation of donepezil hydrochloride polymorph I

5 g donepezil hydrochloride were suspended in methanol (30 ml) and 0.8 ml of water, heated to 60-65°C to get a clear solution. Then this hot solution was slowly added to cooled (0-5°C) isopropyl acetate (60 ml) maintaining the temperature at 5-10°C. The suspension was stirred for another half an hour to one hour.

The product was filtered and dried in vacuum (30-50 mbar) at a temperature of 30-35°C. The drying was controlled by measuring the water content as described in Example 8 and the loss on drying. The yield was from 1.8 to 4.9 g.

### Example 11: Crystallization of donepezil hydrochloride form IV

1 g donepezil hydrochloride was suspended in isopropanol (6 ml) and 1 ml of water, heated to reflux to get a clear solution. Then this hot solution was slowly added to cooled (0-5°C) diisopropyl ether (12 ml) maintaining the temperature at 5-10°C. The suspension was stirred for another half an hour to one hour. The product was filtered and dried in vacuum (30-50 mbar) at a temperature of 30-35°C. The drying was controlled by measuring the water content as described in Example 8 and the loss on drying to get 0.98 g donepezil hydrochloride of form IV.

### Example 12: Preparation of donepezil hydrochloride polymorph I

4.98 kg of donepezil base were suspended at room temperature (20 -25 °C) in methanol (30 1) and heated to 60-65 °C to get a clear solution. The solution was cooled to 30 °C, filtered through a 1 µm filtration cartridge and cooled to 20-25 °C. 1.05 1 of conc. hydrochloric acid were added at 25-30 °C with measuring the pH (3.2 ± 0.2). The solution of donepezil hydrochloride was warmed to 40-45 °C and slowly added to cooled (0-5 °C) diisopropyl ether (60 1) maintaining the temperature at 5-10 °C. The suspension was stirred for another half an hour at 5-10°C. Then, the product was filtered off to obtain 8.45 kg of a wet substance. The wet donepezil hydrochloride hydrate (57 %) was dried in vacuum (30-50 mbar) using the following procedure: Firstly, the product was dried at 25 °C under a slight stream of nitrogen. When it became possible the product was granulated through a sieve of 3x3 mm and through a sieve of 1.2x1.2 mm (in process control, loss on drying LOD 24.3 %). The temperature of drying was raised to 30-35 °C when the LOD was 5.1 %. The drying was also controlled by measuring the assay of water. One hour later the assay of water was 2.77 % and LOD was 3.60 %. The drying was stopped and the substance was exposed to humid air (relative humidity under 60 %) to achieve an assay of water of 4.34 % (Karl Fischer). The yield of the dried product was 4.9 kg.

### Example 13: Crystallization of donepezil hydrochloride polymorph I

10.2 kg of donepezil hydrochloride were suspended in 62 1 of methanol and 1.6 1 of water and heated to 60-65 °C to get a clear solution. The solution was cooled to 50 °C, filtered through a 1 µm filtration cartridge and cooled to 40-45 °C. Then this hot solution was slowly added to cooled (0-5°C) diisopropyl ether (122 1) maintaining the temperature at 5-10°C. The suspension was stirred for another half an hour at 5-10 °C. The product was filtered to obtain 15.9 kg of wet substance. The wet donepezil hydrochloride hydrate (58 %) was dried in vacuum (30-50) mbar using the following procedure: Firstly, the product was dried 1 h at 25 °C under a slight stream of nitrogen. Then the product was granulated through a sieve of 3x3 mm (in process control, loss on drying (LOD) 21.4 %). The drying was continued sieving through a sieve of 1.2x1.2 mm every hour. The temperature of drying was raised to 30 - 35 °C when LOD was 5.8 %. The drying was also controlled by measuring the assay of water. After two hours drying at 30°C the assay of water was 3.66 % and LOD was 3.75 %. The drying was stopped and the substance was exposed to humid air (relative humidity under 60 %) to achieve an assay of water of 4.14 % (Karl Fischer). The yield of the dried product was 9.2 kg.

Alternatively, vacuum filter dryer, rotary vacuum dryer or air dryer can be used for drying donepezil hydrochloride hydrate.

Furthermore, wet donepezil hydrochloride hydrate having an assay of 50-70 % can also be dried under vacuum or via an air fluide bed dryer using one or more of the following steps:
- drying of donepezil hydrochloride hydrate to have donepezil hydrochloride hydrate comprising 30-50 % by weight of solvents,
- drying with stirring or granulating every qualified period (e.g. every hour),
- drying at 20 - 25 °C until loss on drying (LOD) is below 6 %,
- drying at 20 - 25 °C under a slight stream of nitrogen,
- drying at 20 - 25 °C under vacuum below 50 mbar,
- drying at 30 - 35 °C until loss on drying (LOD) is below 4.8 %,
- exposing to humid air (relative humidity under 60 %) when the assay of water is below 4.1 % to achieve an assay of water not more than 5.0 % (Karl Fischer).

## Claims

1. Solid pharmaceutical composition comprising donepezil hydrochloride in the form of a hydrate and excipients, and having a water content of 4 to 10 % by weight (determined by Karl Fischer).

2. Composition according to claim 1, wherein the average particle size of the donepezil hydrochloride is 5 to 300 µm, preferably 10 to 150 µm.

3. Composition according to claim 1 or 2, wherein the particle sizes of the excipients are within the range of D90 < 500 µm, preferably D90 < 350 µm.

4. Composition according to any one of claims 1 to 3 having a water content of 4 to 7 % by weight (determined by Karl Fischer).

5. Composition according to any one of claims 1 to 4, wherein the donepezil hydrochloride is donepezil hydrochloride monohydrate.

6. Composition according to any one of claims 1 to 5, wherein the donepezil hydrochloride is of crystalline form.

7. Composition according to claim 6 wherein the donepezil hydrochloride is donepezil hydrochloride of polymorphic form I or IV.

8. Composition according to any one of claims 1 to 7, wherein the excipients include at least one diluent selected from microcrystalline cellulose and lactose monohydrate.

9. Composition according to any one of claims 1 to 8 wherein the excipients include at least one binder selected from hydroxypropyl cellulose, low-substituted hydroxypropyl cellulose, hydroxypropylmethyl cellulose, starch, and pregelatinised starch.

10. Composition according to any one of claims 1 to 9, wherein the excipients include at least one disintegrant selected from starch, crospovidone, and low-substituted hydroxypropyl cellulose.

11. Composition according to any one of claims 1 to 10 comprising as excipient:
(A) 1 to 50 % by weight of donepezil hydrochloride,
(B) 1 to 90 % by weight of diluent,
(C) 1 to 90 % by weight of binder,
(D) 1 to 40 % by weight of disintegrant, and optionally
(E) 0.1 to 10 % by weight of lubricant.

12. Composition according to any one of claims 1 to 11 wherein the water content of the donepezil hydrochloride hydrate and the various excipients used in the composition is adjusted in such a way that a migration of water from the excipients to the donepezil hydrochloride or vice versa is prevented.

13. Composition according to claim 12 comprising:
(a) donepezil hydrochloride, and
(b) excipents, which are present in the composition in the amount of more than 11%, preferably more than 15 %, more preferably more than 20 % based on the total composition weight, and
(c) excipients, which are present in the composition in the amount of less than 11 %, preferably less than 15%, more preferably less than 20% based on the total composition weight,
wherein the water content of excipients (b), in % by weight, minus the water content of active ingredient (a), in % by weight, is less than 4.0 % (by weight), preferably less than 3.0 % (by weight), most preferably less than 2.0 % (by weight) (determined by Karl Fischer).

14. Composition according to any one of claims 1 to 13, which is in the form of a coated or uncoated tablet, a capsule, pellets, a powder mixture, a granulate or mini tablets filled in capsules.

15. Process for the preparation of the composition according to any one of claims 1 to 14 comprising mixing and processing donepezil hydrochloride and excipients to the desired composition.

16. Process according to claim 15 comprising wetting a part of or all excipients with water.

17. Process according to claim 15 comprising:
(i) granulating a mixture of excipients using water as granulation liquid to give a granulate,
(ii) adding donepezil hydrochloride and excipients to the granulate to give a compression mixture,
(iii) compressing the compression mixture to the desired form, and
(iv) optionally applying a coating.

18. Process according to claim 15 comprising:
(i') granulating a mixture of donepezil hydrochloride and excipients using water as granulation liquid to give a granulate,
(ii') adding excipients to the granulate to give a compression mixture,
(iii') compressing the compression mixture to the desired form, and
(iv') optionally applying a coating.

19. Process according to claim 17 or 18, wherein the temperature of the mixture and of the granulate does not exceed 50°C during the granulating step.

20. Process according to any one of claims 17 to 19, wherein the water content of the granulate is 0.5 to 2.5 %, preferably 1.0 to 2.0 % determined as loss on drying at 85°C, 20 minutes.

21. Process according to any one of claims 17 to 20, wherein the water content of the compression mixture is 1.0 to 6.0 %, preferably 1.5 to 5.0 % determined as loss on drying at 85°C, 20 minutes.

22. Process according to claim 15 comprising
(i') mixing donepezil hydrochloride and excipients to give a compression mixture,
(ii') compressing the obtained compression mixture to the desired form, and
(iii') optionally applying a coating.
